# EUROPÄISCHE PATENTSCHRIFT

(11) **EP 2 648 784 B1**
(45) Veröffentlichungstag und Bekanntmachung des Hinweises auf die Patenterteilung: **25.02.2015**
(21) Anmeldenummer: 11790620.6
(22) Anmeldetag: 01.12.2011
(51) Int. Cl.: A61M 5/31, B65D 55/02, A61M 39/04

(54) **SPRITZENKAPPE**
SYRINGE CAP
CAPUCHON DE SERINGUE

(30) Priorität: 07.12.2010 DE 102010061061
(43) Veröffentlichungstag der Anmeldung: 16.10.2013
(73) Patentinhaber: SCHOTT Schweiz AG, 9001 St. Gallen (CH)
(72) Erfinder: Von Schuckmann, Alfred, 47627 Kevelaer (DE)
(74) Vertreter: Müller, Enno
(86) Internationale Anmeldenummer: PCT/EP2011/071471
(87) Internationale Veröffentlichungsnummer: WO 2012/076388

(56) Entgegenhaltungen:
- WO-A1-2009/028946
- DE-A1- 19 956 243
- FR-A- 782 818
- US-A1- 2008 097 386
- US-B1- 6 193 688
- US-B1- 6 585 691

## Beschreibung

Die Erfindung betrifft eine Spritzenkappe für eine medizinische Spritze, mit zwei zueinander beweglichen Kappenteilen, wobei eine Bewegung der Kappenteile zueinander bei einem ersten Abnehmen der Spritzenkappe von der Spritze erfolgt und hierbei eine Versetzung eines Anzeigeelementes in eine Anzeigestellung erfolgt.

Derartige Spritzenkappen, die auch als Spritzenendkappen oder Tip-Caps bezeichnet werden, sind in vielfältiger Hinsicht bekannt. Beispielsweise ist auf einen Stand der Technik gemäß US 6196998 B1 zu verweisen. Bei der hieraus bekannten Spritzenkappe sind zwei Kappenteile ausgebildet, ein erstes Kappenteil, das einen distalen Endbereich der Spritzenkappe bildet und ein zweites, das einen proximalen Endbereich der Spritzenkappe bildet. "Proximal" bedeutet hierbei, dass es nach dem Aufbringen der Spritzenkappe auf die Spritze zugewandt dem ein Kolbenteil aufweisenden zylindrischen Teil des Spritzenkörpers angeordnet ist. Diese Kappenteile sind über Sollbruchstege miteinander verbunden. Im Zuge eines ersten Abschraubens der Spritzenkappe von der Spritze wird das den distalen Endbereich bildende Kappenteil von dem weiteren Kappenteil durch Abreißen getrennt. Hierdurch wird ein Erstgebrauch erkennbar. Das dem distalen Ende zugeordnete Kappenteil kann von der Spritze abgenommen werden, während das andere Kappenteil an der Spritze verbleibt.

Aus der US 6,585,691 B1 ist eine weitere Spritzenkappe bekannt, bei welcher die Bewegung der Kappenteile zueinander zum Abtrennen einer Hülse führt, die danach zwischen dem Hals des Spritzenkörpers und dem zweiten Kappenteil gefangen ist. Sie kann nach einer Abschraubung des zweiten Kappenteils auch vollständig abfallen.

Ausgehend von dem dargestellten Stand der Technik beschäftigt sich die Erfindung mit der technischen Problematik, eine Spritzenkappe für eine medizinische Spritze anzugeben, bei der bei einfacher Handhabung eine vorteilhafte Erkennbarkeit des Erstgebrauches gegeben ist.

Diese technische Problematik ist bei einer Spritzenkappe gelöst, bei welcher das Anzeigeelement zusammen mit den Kappenteilen der Spritzenkappe von der Spritze abnehmbar ist. Das Anzeigeelement ist an dem insgesamt von der Spritze abnehmbaren Teil ausgebildet. Es braucht kein Teil an der Spritze zu verbleiben.

Bevorzugt ist zunächst vorgesehen, dass sich das Anzeigeelement an der distalen Seite der Spritzenkappe befindet. Es ist also bevorzugt dem äußeren Ende der Spritzenkappe zugeordnet. Eine gute Sichtbarkeit ist hierdurch schon gewährleistet.

Weiterhin ist bevorzugt, dass an einem Kappenteil ein oder mehrere Gewindegänge ausgebildet sind, zur Zusammenwirkung mit einem ein Gegengewinde aufweisenden Halterungsteil. Das Halterungsteil ist bevorzugt ein einen Luer-Anschluss an der Spritze umgebender Halterungskragen. Hierbei ist auch vorgesehen, dass der eine oder jedenfalls einer der an dem Kappenteil ausgebildeten Gewindegänge oder eine Nutausformung zwischen Abschnitten des Gewindeganges eine Bremsausformung aufweist. Dadurch, dass ein Gewindegang eine Bremsausformung aufweist, ist dem Abschrauben der Spritzenkappe ein gewisser Widerstand entgegengesetzt. Dieser Widerstand kann verhindern helfen, dass eine Spritzenkappe ungewollt abgeschraubt wird. Er kann aber auch, wie nachstehend noch erläutert, dazu benutzt sein, das Anzeigeelement in die Anzeigestellung zu versetzen.

Insbesondere ist bevorzugt, dass die Kappenteile zunächst relativ zueinander verdrehbar sind und am Ende Ihrer relativen Verdrehung in eine eine Drehkopplung ergebende Verrastungsstellung überführbar sind. Insbesondere im Hinblick auf diese Überführbarkeit in die Verrastungsstellung ist die Bremsausformung an dem Gewindegang günstig. Hierdurch kann eingestellt sein, dass bei einem Abdrehen der Spritzenkappe zuverlässig zunächst die Überführung der Kappenteile in die Drehkopplung erfolgt und dann erst die Abschraubung der Spritzenkappe von der Spritze sich bei einer weiteren Schraub-Betätigung vollzieht.

In weiterer Einzelheit ist bevorzugt, dass die Außenkappe ein erstes bewegliches Kappenteil ist und dass innerhalb der Außenkappe ein Innenkappenteil angeordnet ist, das den Gewindegang aufweist. Das Außenkappenteil ist hierbei relativ zu dem Innenkappenteil beweglich, insbesondere drehbeweglich, vorgesehen. Der an dem Innenkappenteil ausgebildete Gewindegang ist gleichwohl bevorzugt einer Außenfläche der Spritzenkappe zugeordnet. Der Gewindegang ist bevorzugt an dem sich unterhalb einer unteren Randkante der Außenkappe erstreckenden Bereich der Innenkappe ausgebildet

Das Innenkappenteil kann in weiterer Einzelheit aus einem Abdichtteil und einem Schraubteil bestehen. Das Abdichtteil ist zur eigentlichen Abdichtung der Mündung der Spritze vorgesehen. Es besteht bevorzugt aus einem Gummi- oder Elastomerwerkstoff. Es kann pressgeformt und vulkanisiert sein oder spritzgegossen. Das Schraubteil und die Außenkappe sind bevorzugt im Kunststoffspritzverfahren hergestellte Hartkunststoffteile. Das Schraubteil erbringt bevorzugt vornehmlich die Schraubverbindung zu dem Spritzenkörper. Das Schraubteil erbringt auch bevorzugt die Halterung des Außenkappenteils an dem Innenkappenteil.

Weiter ist bevorzugt, dass das Abdichtteil einen Teil der Außenfläche des Schraubteils bildet. Hierzu kann beispielsweise eine Durchsetzung von Teilbereichen des Schraubteils durch das Abdichtteil, beispielsweise ein oder mehrerer diesbezüglicher Öffnungen des Schraubteils, vorgesehen sein, so dass die Außenfläche des Schraubteils teilweise durch das Abdichtteil gebildet ist. Insbesondere im Bereich eines Gewindeganges oder zwischen zwei Gewindegängen. Da das Abdichtteil bevorzugt aus einem Gummi- und/oder Elastomerwerkstoff besteht, kann es so die Funktion des Bremselementes an dem Innenkappenteil übernehmen. Bevorzugt ist auch vorgesehen, dass es hierzu mit einer geringfügig größeren Erstreckung, insbesondere in radialer Richtung, ausgebildet ist. Insbesondere ist vorgesehen, dass an dem Abdichtteil zur Ausbildung des Bremsteils ein oder mehrere sich axial erstreckende Formschlussausformungen ausgebildet sind. Diese Formschlussausformungen sind eingepasst in entsprechende Formschlussausnehmungen des Schraubteils. Die Formschlussausnehmungen des Schraubteils können insbesondere von dessen proximalem Ende ausgehende Durchbrüche sein, die sich im Wesentlichen axial erstrecken.

Weiter ist bevorzugt, dass an einem der Kappenteile, bevorzugt an dem Innenkappenteil, weiter bevorzugt an dem Schraubteil, eine Auflauframpe ausgebildet ist, mit welcher bei dem ersten Verdrehen der Kappenteile im Zuge eines Abschraubens der Spritzenkappe von der Spritze das Anzeigeelement in die Anzeigestellung bewegt wird. Bevorzugt wird das Anzeigeelement auch unter Zerstörung von Abreißstegen in die Anzeigestellung hierbei bewegt. Das Anzeigeelement oder ein auf das Anzeigeelement einwirkendes Teil kann durch Auflaufen auf die Auflauframpe im Zuge des Verdrehens der Kappenteile zueinander bewegt werden.

Hinsichtlich des Halterungsteils ist einerseits bevorzugt, dass es unmittelbar an die Spritze angeformt ist. Dies ist insbesondere bei vollständig im Kunststoffspritzverfahren hergestellten Spritzenkörpern der Fall. Es handelt sich in der Regel um eine kragenförmige Ausbildung mit einem Innengewinde, umgebend zu dem Luer-Anschluss.

Alternativ kann die hier beschriebene Spritzenkappe aber auch für Spritzen, insbesondere Glasspritzen, vorgesehen sein, bei welchen kein Halterungskragen mit entsprechendem Gewinde angeformt ist. Hierzu ist dann bevorzugt vorgesehen, dass die hier beschriebene Spritzenkappe zusätzlich mit einem Halterungsteil an ihrem proximalen Ende ausgebildet ist. Das Halterungsteil ist weiter bevorzugt mit einer zentralen Durchgangsöffnung ausgebildet, zur Verbindung mit dem Hals, vorzugsweise im Bereich des Luer-Anschlusses, eines Spritzenkörpers. Die Verbindung kann eine Klemm- und/oder Formschlussverbindung sein. Beim Abschrauben der Spritzenkappe von der Spritze verbleibt das Halterungsteil an der Spritze. Zur Befestigung mit der Spritze kann es auf diese mittels Druck, der zu einer entsprechenden elastischen Aufweitung und dann dem gewünschten Klemmsitz führt, aufgesetzt werden.

Nachstehend ist die Erfindung des Weiteren anhand der beigefügten Zeichnungen, die jedoch lediglich ein Ausführungsbeispiel darstellen, erläutert. Hierbei zeigt:
- Fig. 1: eine Seitenansicht einer lediglich angedeuteten Spritze mit einer Spritzenkappe;
- Fig. 2: eine Draufsicht auf den Gegenstand gemäß Figur 1;
- Fig. 3: eine perspektivische Ansicht von schräg oben der Spritzenkappe;
- Fig. 4: eine perspektivische Ansicht von schräg unten der Spritzenkappe;
- Fig. 5: eine Explosionsdarstellung der Spritzenkappe und des zugeordneten Endes der Spritze, in einer Ansicht von schräg oben;
- Fig. 6: eine Darstellung gemäß Figur 5, in einer Ansicht von schräg unten;
- Fig. 7: einen Querschnitt durch eine auf eine Spritze mit angeformter Halterungsausformung aufgesetzten Spritzenkappe, vor einem erstmaligen Abschrauben und beschränkt auf den oberen Bereich der Spritze;
- Fig. 8: eine Darstellung gemäß Figur 7, mit einer um neunzig Grad versetzten Schnittebene;
- Fig. 9: einen Querschnitt durch Figur 8, geschnitten entlang der Linie IX - IX;
- Fig. 10: eine perspektivische Ansicht von schräg oben der Spritzenkappe nach einem erstmaligen Abschrauben von der Spritze und mit in die Anzeigestellung versetztem Anzeigeelement;
- Fig. 11: eine Draufsicht auf den Gegenstand gemäß Figur 10;
- Fig. 12: einen Querschnitt durch den Gegenstand gemäß Figur 11, geschnitten entlang der Linie XII - XII;
- Fig. 13: einen Querschnitt durch den Gegenstand gemäß Figur 12, geschnitten entlang der Linie XIII - XIII;
- Fig. 14: einen Querschnitt durch den Spritzenkörper nach Abnahme der Verschlusskappe;
- Fig. 15: eine Seitenansicht eines weiteren, angedeuteten Spritzenkörpers mit einer Spritzenkappe zweiter Ausführungsform;
- Fig. 16: eine Draufsicht auf den Gegenstand gemäß Figur 15;
- Fig. 17: den Spritzenkörper zweiter Ausführungsform in einer perspektivischen Ansicht von schräg oben;
- Fig. 18: den Spritzenkörper zweiter Ausführungsform in einer perspektivischen Ansicht von schräg unten;
- Fig. 19: eine Explosionsdarstellung des Spritzenkörpers zweiter Ausführungsform in einer perspektivischen Ansicht von schräg oben;
- Fig. 20: eine Darstellung gemäß Figur 19, in einer perspektivischen Ansicht von schräg unten;
- Fig. 21: eine Querschnittsdarstellung des Spritzenkörpers zweiter Ausführungsform, aufgesetzt auf eine Spritze, vor einem ersten Abschrauben der Spritzenkappe;
- Fig. 22: einen Querschnitt gemäß Figur 21, in einer um neunzig Grad versetzten Querschnittsebene;
- Fig. 23: einen Querschnitt durch den Gegenstand gemäß Figur 22, geschnitten entlang der Linie XXIII - XXIII;
- Fig. 24: eine Darstellung gemäß Figur 10 der Spritzenkappe zweiter Ausführungsform;
- Fig. 25: eine Draufsicht auf den Gegenstand gemäß Figur 24;
- Fig. 26: eine Darstellung der Spritzenkappe zweiter Ausführungsform nach einer ersten Betätigung aber vor dem Abschrauben von der Spritzenkappe;
- Fig. 27: eine Draufsicht auf den Gegenstand gemäß Figur 26 und
- Fig. 28: eine Querschnittsansicht der Spritze nach abgeschraubter Spritzenkappe zweiter Ausführungsform.

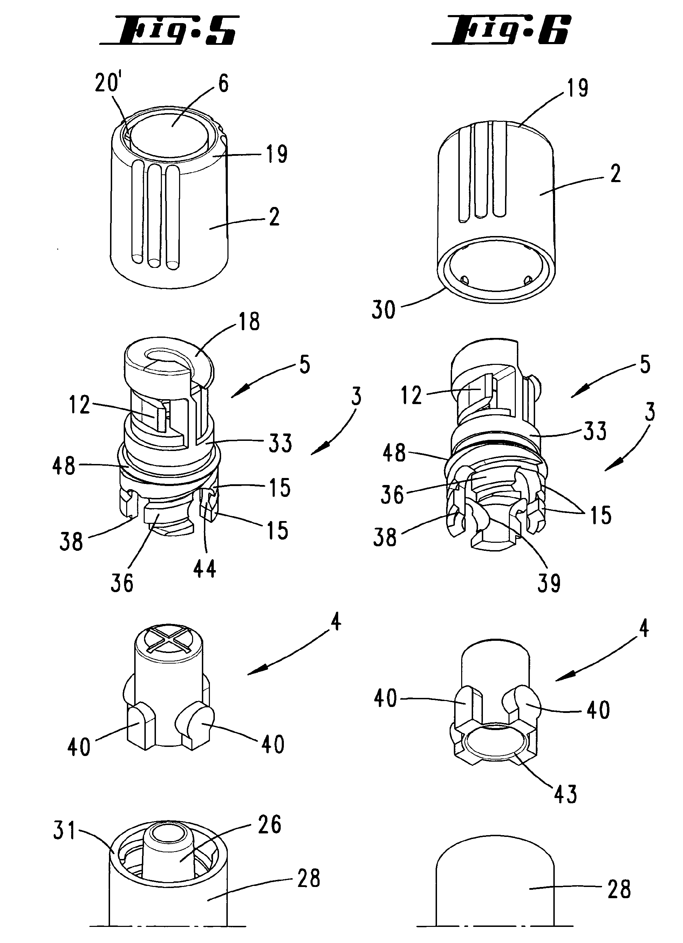

Dargestellt und beschrieben ist zunächst eine Spritzenkappe 1 erster Ausführungsform, die, wie insbesondere mit Bezug zu den Figuren 5 und 6 ersichtlich ist, aus einer Außenkappe 2 und einer Innenkappe 3 besteht, wobei bevorzugt beim Ausführungsbeispiel die Innenkappe 3 sich aus einem Abdichtteil 4 und einem Schraubteil 5 zusammensetzt.

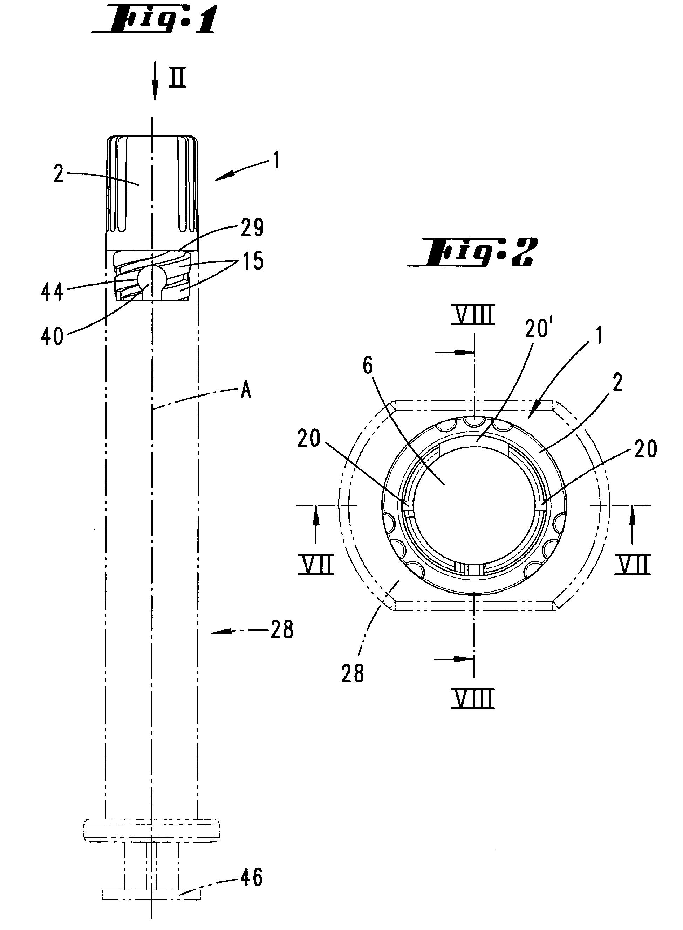

Wie aus Figur 1 ersichtlich, bildet die im aufgesetzten Zustand allein sichtbare Außenkappe 2 bezüglich der Spritze 28 das distale Ende des Verbundes von Spritzenkappe 1 und Spritze 28. Mit "distal" ist ein einem ein Kolbenteil 35 aufnehmenden zylindrischen Teil 34 des Spritzenkörpers abgewandter Endbereich angesprochen. Es ergibt sich im diesbezüglichen Endbereich der Spritze das Aussehen einer Zweiteiligkeit, mit einer rechtwinklig zu einer Längsachse A der Spritze 28 (wie auch der Spritzenkappe 1) verlaufenden Trennfuge 29. Die Trennfuge 29 ergibt sich durch die Unterkante 30 der Außenkappe 2 und die Oberkante 31 der Halterung 17. Die Außenkappe 2 ist ersichtlich in Längsrichtung der Spritze 28 fluchtend zu der Kontur der Spritze 28 ausgebildet.

Das Kolbenteil 35 ist in üblicher Weise mittels einer Handhabe 46 in der Spritze 28 längs beweglich.

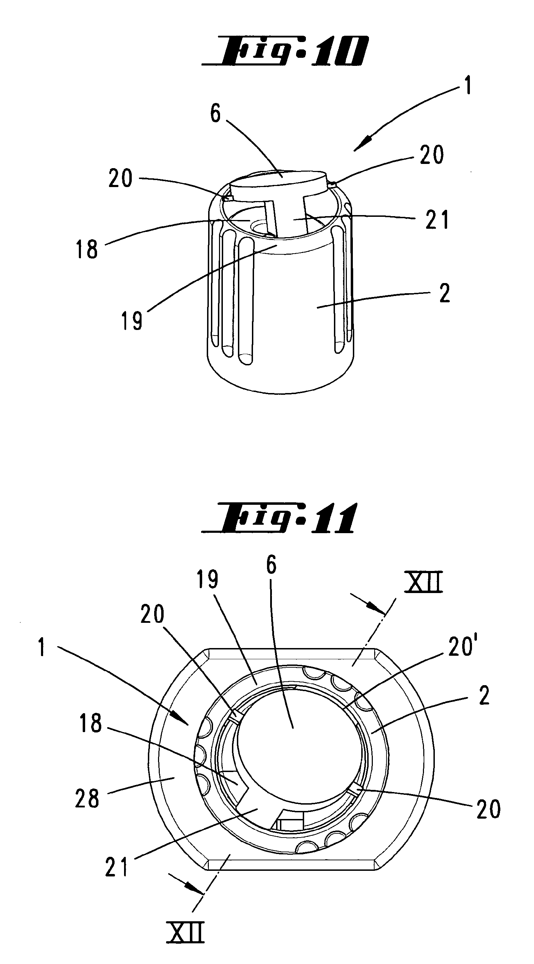

In der Außenkappe 2 ist ein Anzeigeelement 6 ausgebildet, das in eine Anzeigestellung, wie beispielsweise in den Figuren 10 und 11 beziehungsweise 24 und 25 dargestellt, versetzbar ist. Vor einem Erstgebrauch ist das Anzeigeelement mittels Abreißstegen, beim Ausführungsbeispiel zwei Abreißstegen 20, die gegenüberliegend angeordnet sind, an der Außenkappe 2 angebunden. Weiter ist ein Filmgelenk 20' ausgebildet. Hierüber bleibt das Anzeigeelement 6 auch in der Anzeigestellung mit der Außenkappe 2 verbunden. Das Anzeigeelement 6 weist in der Draufsicht eine runde, vorzugsweise kreisförmige Kontur auf. Die Außenkappe 2 ist weiter bevorzugt als fingerhutartige Hülse gestaltet, wobei das Anzeigeelement 6 die Stirnfläche oder einen wesentlichen Teil der Stirnfläche bildet.

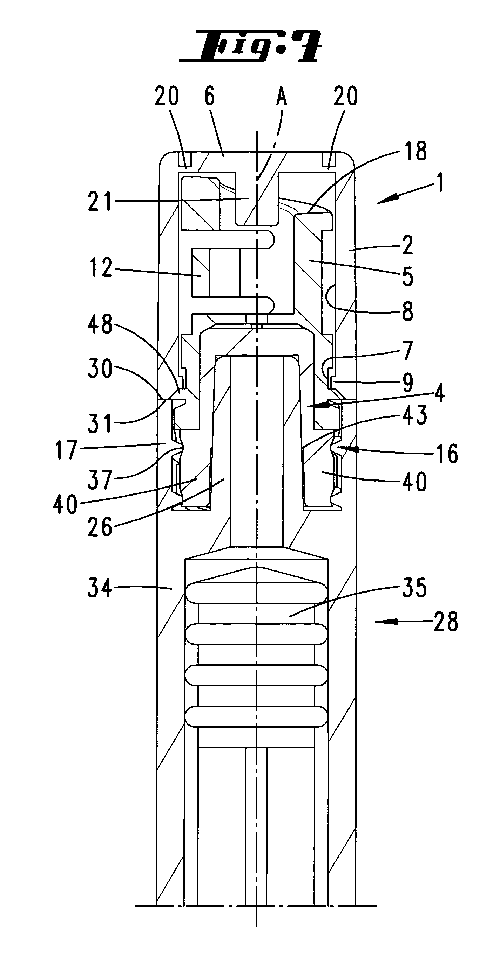

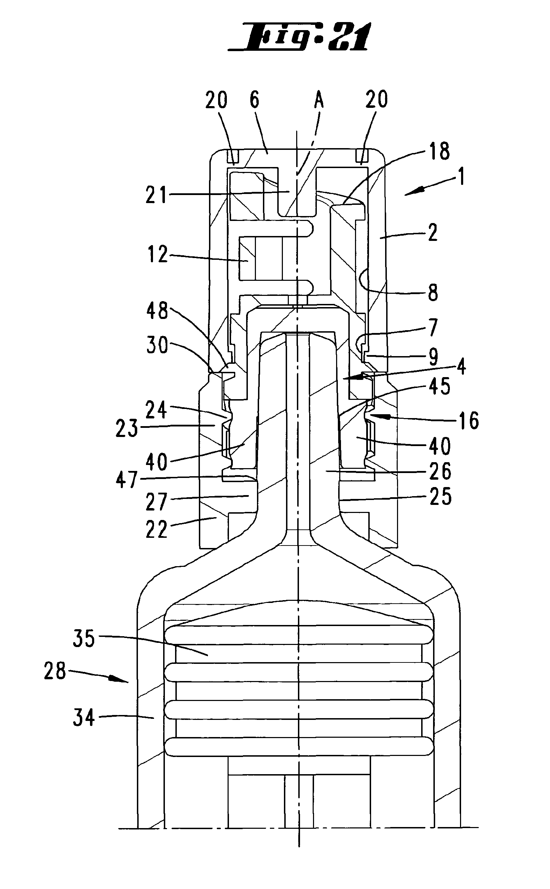

Die Außenkappe 2 und die Innenkappe 3 bilden zwei Kappenteile, die zueinander bewegbar, nämlich drehbewegbar, sind. Hierzu ist im Einzelnen das Außenkappenteil 2, wie etwa aus den Figuren 7 und 21 ersichtlich, in einer Formschlussausnehmung 7 der Innenkappe 3, bevorzugt des Schraubteils 5, aufgenommen. Es weist hierzu einen gegenüber einer Innenfläche 8 vorstehenden Eingriffsvorsprung 9 auf, der in die Ausnehmung 7 eingreift.

Die Außenkappe 2 ist bevorzugt, und beim Ausführungsbeispiel auch nur, um eine Drehachse relativ zu dem Innenkappenteil 3, beziehungsweise konkret bevorzugt dem Schraubteil 5, drehbewegbar, die mit der Längsachse A der Spritzenkappe zusammenfällt Weiter ist die Drehbewegbarkeit bevorzugt derart ausgebildet, dass sich im Zuge der Drehung des Außenkappenteils 2 relativ zu der Innenkappe 3 keine teleskopierende Bewegung ergibt Vielmehr ergibt sich für einen gegebenen Punkt des Außenkappenteils 2 nur eine Bewegung in einer sich quer zu der Längsachse A der Spritzenkappe erstreckenden Ebene, wobei weiter bevorzugt die Ebene rechtwinklig zu der Längsachse A verläuft. Die Drehbewegung ist auch bevorzugt nur über einen Winkelbereich von weniger als 360° ermöglicht.

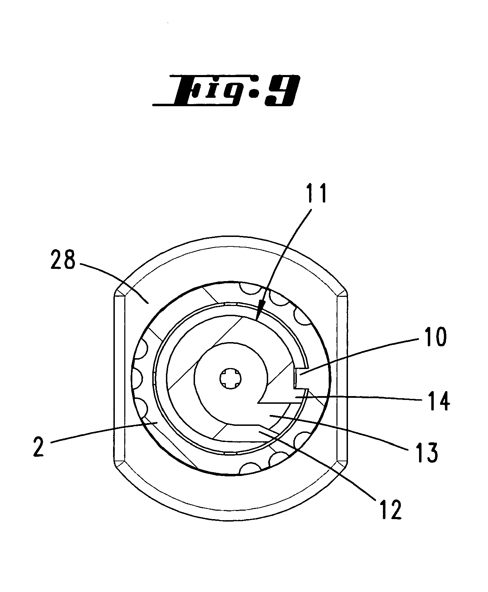

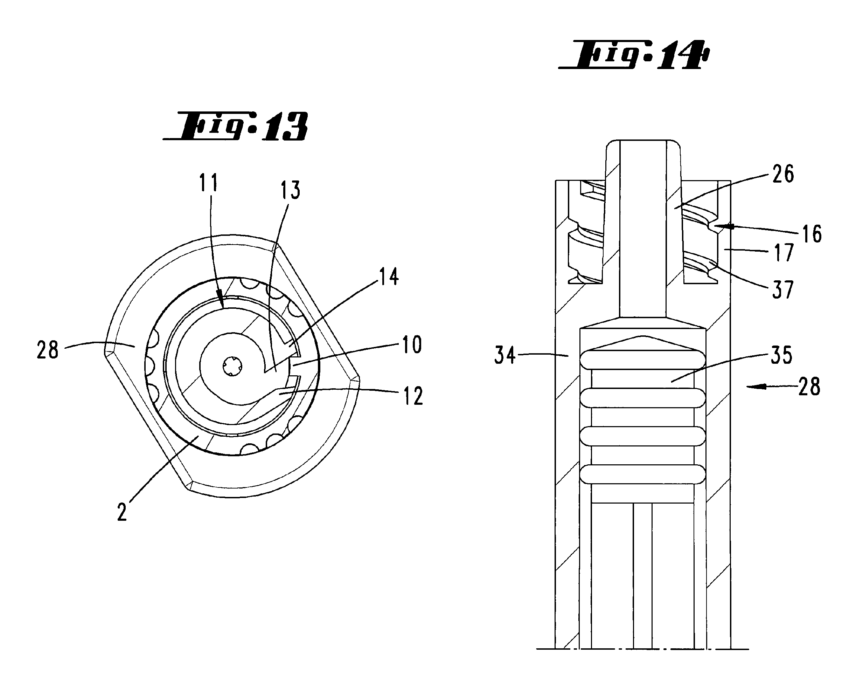

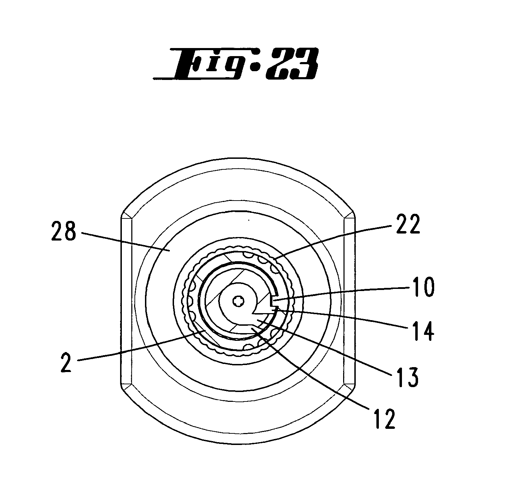

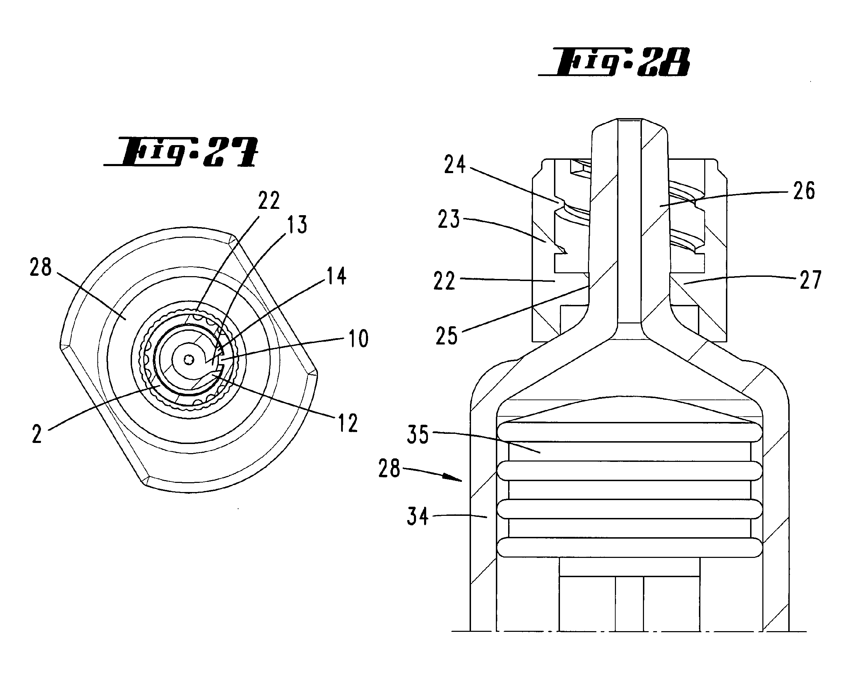

Die Außenkappe 2 weist weiter, wie etwa aus den Figuren 9 und 23 zu entnehmen, einen Rastvorsprung 10 auf, der sich axial erstreckt. Der Rastvorsprung 10 wirkt zunächst, mittels seiner Stirnseite, mit einem stufenartigen Verjüngungsabschnitt 11 der Innenkappe 3 beziehungsweise bevorzugt des Schraubteils 5 zusammen. Der Rastvorsprung 10 trägt weiter bevorzugt, jedenfalls bei der Drehbewegung der Außenkappe 2 relativ zu der Innenkappe 3, aufgrund einer entsprechenden axialen Erstreckung zu einer stabilen Führung der Außenkappe 2 an der Innenkappe 3 bei. Die Wandung des stufenartigen Verjüngungsabschnittes 11 läuft, wie aus Figur 9 auch ersichtlich, in Umfangsrichtung in eine nach radial außen vorstehende Rastausformung 12 aus, die ein freistehendes Ende aufweist. Beim Verdrehen der Außenkappe 2 relativ zu der Innenkappe 3 beziehungsweise dem Schraubteil 5 überläuft der Vorsprung 10 unter elastischer Zurückdrückung nach radial innen die Rastausformung 12 bis der Rastvorsprung 10 in Überdeckung zu einem - in Umfangsrichtung gesehen - Abstandsbereich 13 zwischen der Ausformung 12 und einer Anschlagwand 14 ist, vergleiche hierzu auch Figuren 13 und 27. Da nach dem Überlaufen die Ausformung 12 elastisch zurückspringt, ist der Vorsprung 10 dann in dem Abstandsbereich 13 gefangen. Somit ist eine Drehkopplung zwischen den Kappenteilen, der Außenkappe 2 und der Innenkappe 3 erreicht. Ein weiteres Drehen der Spritzenkappe 2 führt zu einem Abschrauben der Spritzenkappe 2 von der Spritze 28.

Diese Drehkopplung ist auch nicht reversibel. Bei einem Wiederaufschrauben der Spritzenkappe auf die Spritze 28 ist also sogleich die Handhabung wie bei einer insofern einteiligen Spritzenkappe gegeben.

Die Innenkappe 3, beziehungsweise bevorzugt das Schraubteil 5 der Innenkappe 3, weist weiter, unterseitig des Rücksprungbereiches 11, weiter bevorzugt unterhalb einer Stufe 33 und/ oder unterhalb der Unterkante 30 der Außenkappe 2, bezogen auf den zusammengesteckten Zustand, einen oder mehrere Außen-Gewindegänge 15 auf. Der Bereich des Außengewindeganges 15 ist, bevorzugt etwa um das Maß einer radialen Dicke einer Halterung 17 nach innen gegenüber dem Außendurchmesser der Außenkappe 2 (im unteren Bereich) zurückversetzt gebildet. Andererseits entspricht der größte Außendurchmesser im Bereich des Gewindeganges etwa dem Außendurchmesser der Innenkappe im Bereich der Stufe 33 oder ist noch etwas größer.

Die Rastausnehmung 7 ist zwischen dem Verjüngungsabschnitt 11 beziehungsweise darüber hinaus bevorzugt der Stufe 33 und dem Gewindegang 15 vorgesehen. Die Rastausnehmung 7 ist auch bevorzugt als Rücksprung ausgebildet. Der in diese Rastausnehmung 7 eingreifende Eingriffsvorsprung 9 ist etwas nach oben versetzt zu dem unteren Rand 30 ausgeformt. Unterhalb der Stufe 33 und oberhalb des Gewindeganges 15 ist weiter bevorzugt an dem Schraubteil 5 ein bevorzugt gegenüber dem Gewindegang 15 und/oder der Stufe 33 radial vorspringender, bevorzugt auch oberseitig abgeschrägt umlaufender Vorsprung 48 ausgebildet. An diesem Vorsprung 48 liegt die Außenkappe innenseitig, im Bereich ihrer Unterkante 30 im zusammengesetzten Zustand bevorzugt an.

Der bevorzugt zylinderförmig gebildete Verjüngungsabschnitt 11 ist nach oben durch einen radial überstehenden, mit seiner Unterkante auch horizontal umlaufenden Vorsprung 32 begrenzt. Insgesamt ist so die axiale Halterung der Außenkappe 2 an der Innenkappe 3 erreicht. Der Vorsprung 32 bildet oberseitig die weiter unten noch näher erläuterte Auflauframpe 18 aus.

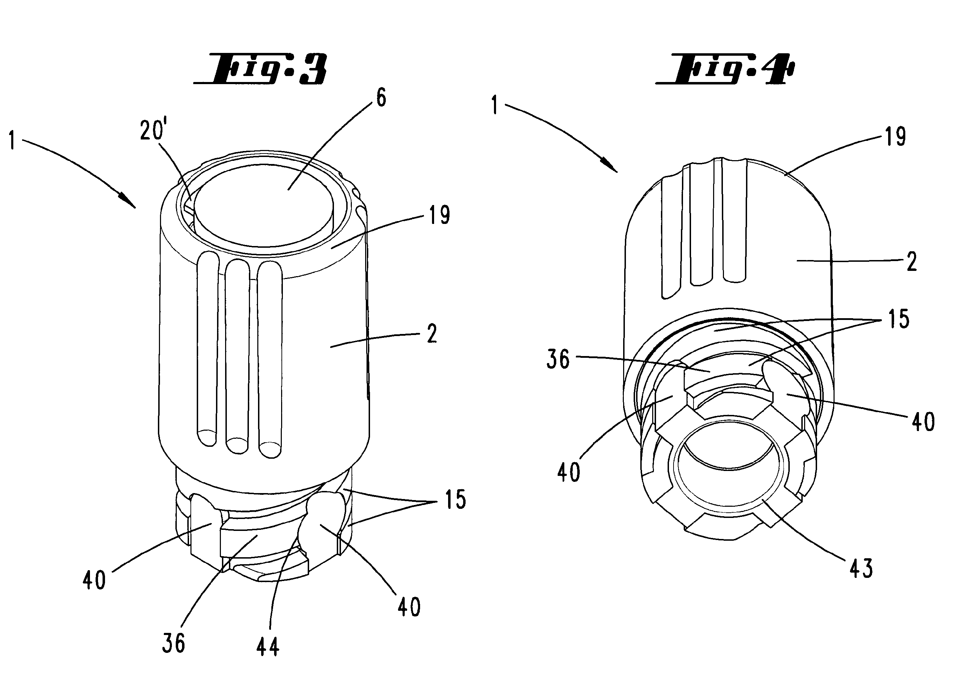

Bezüglich eines Gewindeganges 15 beziehungsweise eines mit einer Nutausformung 36 zwischen zwei axial übereinander befindlichen Abschnitten des Gewindegangs 15 zusammenwirkenden Innengewindeganges 37 (vergleiche auch Figur 14) der Halterung 17 ist eine Bremsausformung vorgesehen. Die Bremsausformung ist beim Ausführungsbeispiel und bevorzugt durch einen Teilbereich des Abdichtteiles 4 gegeben, das durch Durchgriffsöffnungen 44 des Schraubteils 5 hindurchgreift. Hierzu ist insbesondere auf die Figuren 3 und 4 zu verweisen. Hierdurch wirkt das Innenkappenteil 3 insgesamt im Bereich der Gewindegänge 15 mit der Halterung 17 der Spritze 28 derart bremsend zusammen, dass bei einem versuchten Abschrauben der Spritzenkappe 1 sich zunächst die beschriebene Verdrehung zwischen der Außenkappe 2 und der Innenkappe 3 ergibt, bis die Außenkappe 2 sich in der genannten Drehverriegelungsstellung befindet, wodurch die Drehkopplung zwischen der Außenkappe 2 und dem Innenkappenteil 3 erreicht ist. Die elastische Bewegbarkeit des im Querschnitt als sich nach außen erweitender Spiralabschnitt gebildeten Rastausformung 12 ist so eingestellt, dass zufolge der genannten Bremswirkung zunächst das beschriebene Überlaufen des Rastabschnittes 12 erfolgt, bevor ein Abschrauben der Spritzenkappe 1 von der Spritze 28 sich einstellt.

Die Durchgriffsöffnungen 44 des Schraubteils 5 sind im Einzelnen als von dem proximalen Ende, einer diesbezüglichen Randkante 38 des Schraubteils 5, ausgehende, sich axial erstreckende Öffnungen ausgebildet.

Bevorzugt sind über den Umfang verteilt in dem Schraubteil 5 Durchgriffsöffnungen 44 mit einem Hinterschnitt 39 und Durchgriffsöffnungen 44 ohne einen Hinterschnitt 39 ausgebildet. Weiter bevorzugt handelt es sich um zwei Durchgriffsöffnungen 44 mit Hinterschnitt in axialer Richtung und zwei Öffnungen ohne Hinterschnitt in axialer Richtung. Die Öffnungen ohne Hinterschnitt sind im Wesentlichen torbogenförmig gestaltet, während die Öffnungen mit Hinterschnitt im Wesentlichen bevorzugt pilzförmig im Grundriss gestaltet sind.

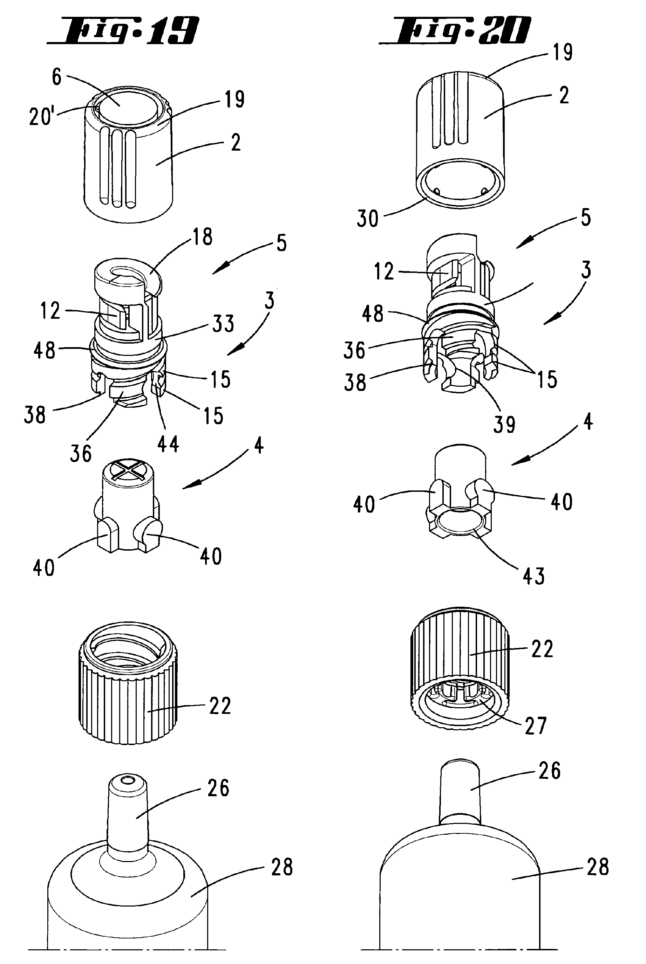

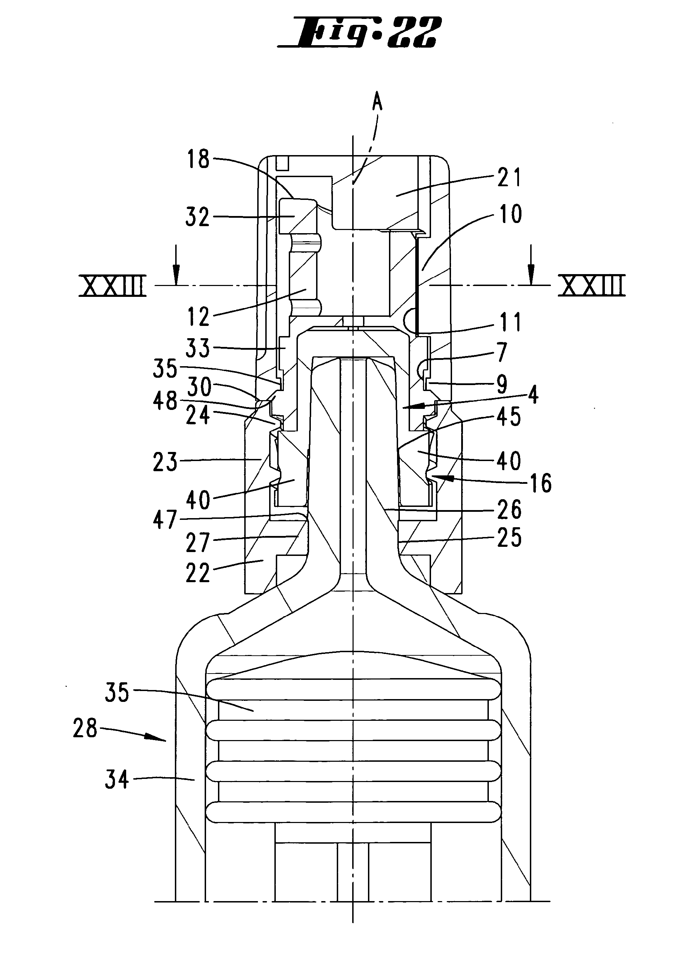

Das Abdichtteil 4 weist entsprechend geformte radial vorstehende Ausformungen 40 auf, vergleiche insbesondere Figuren 5, 6 und 19, 20, die auch entsprechend dem torbogenförmigen Grundriss beziehungsweise dem pilzförmigen Grundriss der Durchgriffsöffnungen 37 geformt sind. Auch diese Ausformungen gehen vom proximalen Ende des Abdichtteils 4 aus. Diese Ausformungen 40 sind im Einzelnen die Bremsausformungen. Hierzu ist bevorzugt vorgesehen, dass sie in die Nutausformung 36 vorstehen. Weiter bevorzugt überragen sie aber radial nicht eine axiale Außenfläche eines Gewindeganges 15. Die sich aufgrund der elastischen Ausbildung der Ausformungen 40 ergebenden Verformungen im Verschraubungszustand sind beispielsweise den Figuren 7 und 8 sowie 21 und 22 zu entnehmen.

Im Übrigen ist das Abdichtteil 4 im Wesentlichen hutförmig gestaltet. Es weist auch bevorzugt einen rotationssymmetrischen Querschnitt auf, mit Ausnahme der genannten Ausformungen 40.

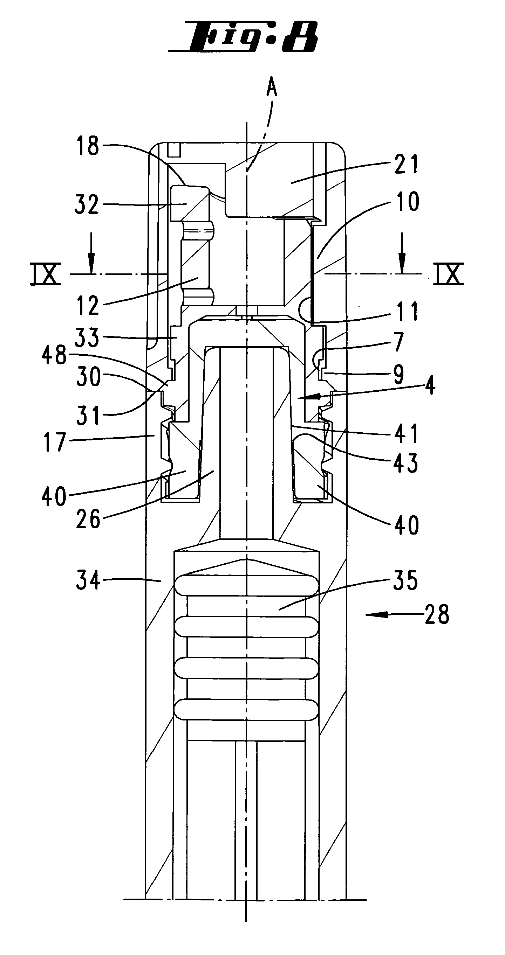

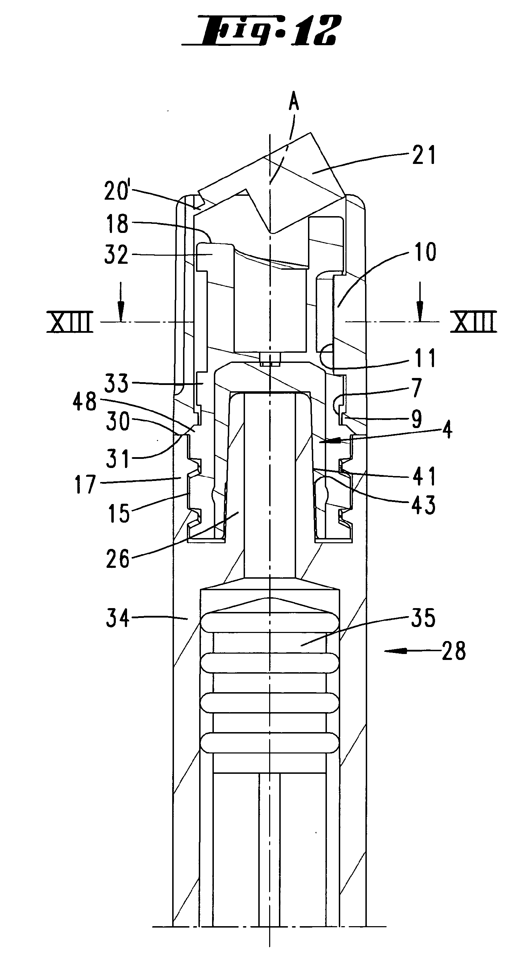

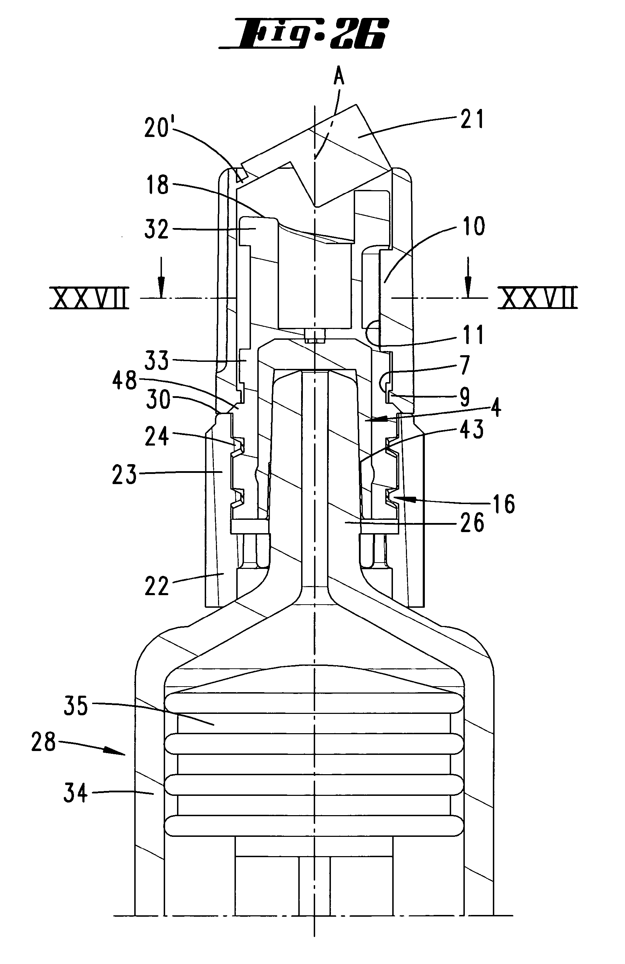

An dem Schraubteil 5 ist weiterhin eine sich im Grundriss kreisförmig aber spiralartig ansteigend erstreckende Auflauframpe 18 ausgebildet (vergleiche etwa Figuren 5 und 8). Mit dieser Auflauframpe 18 wirkt beim Verdrehen der Außenkappe 2 relativ zu der Innenkappe 3 das Anzeigeelement 6 zusammen. Die Zusammenwirkung ist bevorzugt derart, dass das Anzeigeelement 6 relativ zu einer Stirnkante 19 der Außenkappe 2 im Zuge der Verdrehung angehoben wird. Hierzu weist das Anzeigeelement 6 bevorzugt unterseitig einen Auflaufnocken 21 auf. Das Anzeigeelement wird hierdurch im Zuge der Drehung der Außenkappe 2, die sich hierbei axial praktisch nicht bewegt, relativ zu der Außenkappe 2 angehoben. Ein oder mehrere der Abreißstege 20, beim Ausführungsbeispiel zwei Abreißstege 20, werden beim Anheben des Anzeigeelementes 6 in die Anzeigestellung gemäß Figur 10 durchtrennt. Die Anzeigestellung ist ersichtlich dadurch gegeben, dass das Anzeigeelement 6 aus einer Stellung mit in etwa rechtwinkliger Erstreckung zur Längsachse A in eine Stellung, im Vertikalquerschnitt, mit einem spitzen Winkel zu der Längsachse A hinsichtlich seiner Haupt-Erstreckungsrichtung verlagert wird. Das verbleibende Filmscharnier 20' (vergleiche auch Figuren 2 und 12 beziehungsweise 16 und 26) bewirkt gleichsam eine Drehhalterung des Anzeigeelementes 6. Das Anzeigeelement 6 ist mit Ausnahme des Auflaufnocken 21 beim Ausführungsbeispiel bevorzugt im Wesentlichen plattenförmig ausgebildet, nämlich wie bevorzugt auch angegeben, als Plattenteil mit kreisförmiger Kontur. Der Auflaufnocken 21 ist bevorzugt als sich radial erstreckende Rippe gebildet. Dieser Auflaufnocken 21 wirkt in weiterer Einzelheit mit der Auflauframpe 18 zusammen. Der angehobene beziehungsweise verdrehte Zustand des Anzeigeelementes 6 kann nicht mehr in die genannte rechtwinklige Erstreckung zurückversetzt werden, da der Auflaufnocken 21 auch im verdrehten End-Zustand (Drehkopplung) von Außenkappe 2 und Innenkappe 3 sich in Überdeckung zu der Auflauframpe 18 befindet. Und zwar bevorzugt in Überdeckung zu einem obersten Bereich der Auflauframpe 18. Siehe hierzu auch Figuren 12 und 26.

Das Abdichtteil 4 weist innenseitig eine Ausnehmung 41 auf, die an den Luer-Vorsprung 42 der Spritze 28 angepasst ist. Diese Ausnehmung 41 ist bis auf die dem proximalen Ende im aufgesetztem Zustand des Abdichtteils 4 ausgebildete Öffnung 43 vollständig geschlossen. Zu dem distalen Ende der Spritzenkappe 1 hin versetzt ist die Ausnehmung 41 bevorzugt mit einem umlaufendem Absatz 45 versehen. Oberhalb des Absatzes 45 setzt sich die Ausnehmung 41, die auch bevorzugt angepasst konisch an die Luer-Ausformung der Spritze gebildet ist, fort. Diese stufenartige Verjüngung der Ausnehmung kann nochmals dazu beitragen, die Abdichtung zu verbessern.

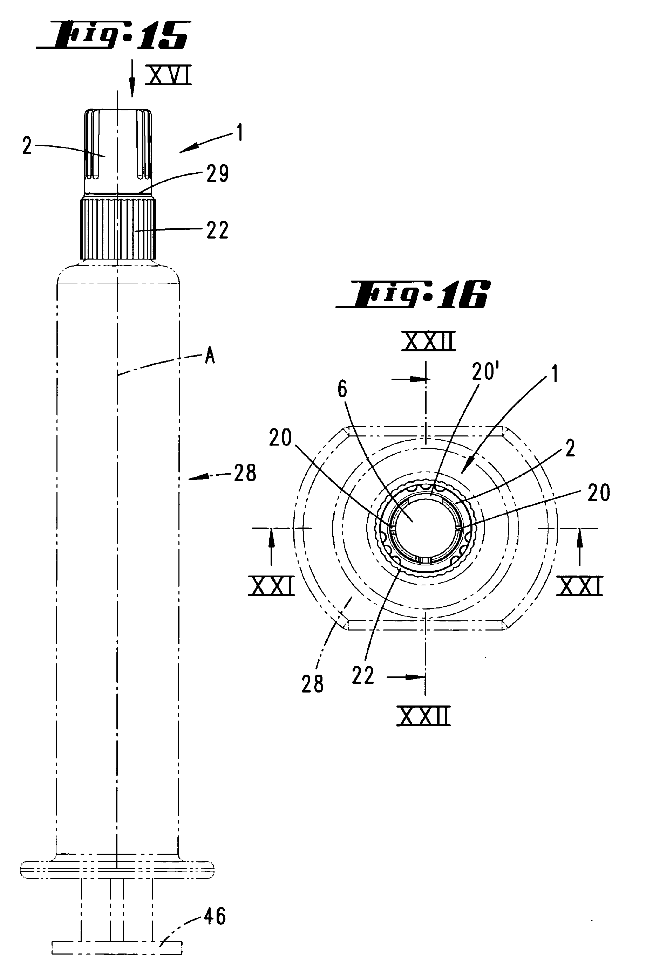

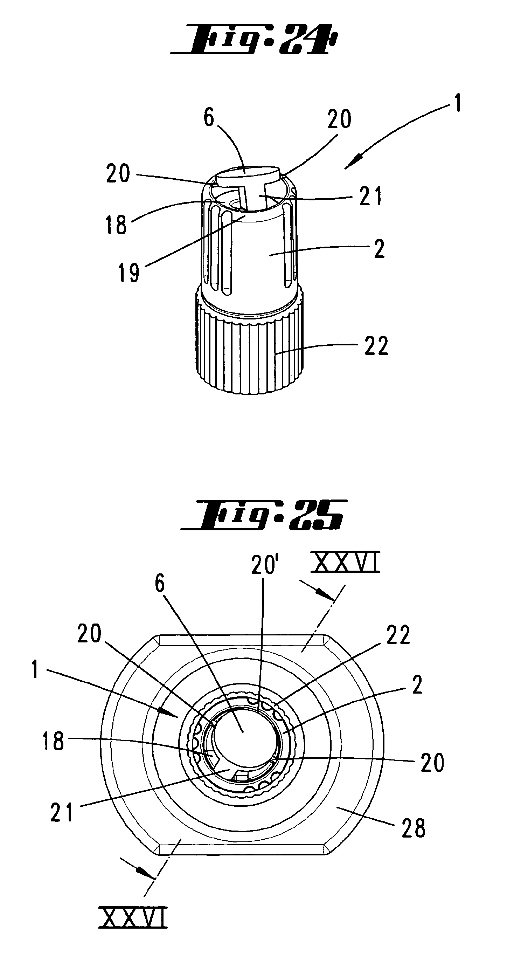

Bezüglich der zweiten Ausführungsform, wie sie in den Figuren 15 bis 28 dargestellt ist, sind gleiche Teile mit gleichen Bezugszeichen versehen. Die diesbezügliche Beschreibung der ersten Ausführungsform ist auch auf die zweite Ausführungsform zutreffend.

Die zweite Ausführungsform betrifft die Ausbildung einer Spritzenkappe 1 für eine Spritze, die nicht mit einer Halterungsausformung 17 mit Innengewinde ausgebildet ist. Dies ist gewöhnlich eine Glasspritze.

Um gleichwohl eine Halterung der Spritzenkappe 1 an der Spritze 28 zu ermöglichen, ist die Spritzenkappe 1 der zweiten Ausführungsform mit einem Halterungsteil 22 ausgebildet Die Spritzenkappe 1, die im Lieferzustand, vor einem ersten Aufbringen auf eine Spritze 28, mit dem Halterungsteil 22 schraubverbunden sein kann, ist nach einer Aufbringung der Kombination von Spritzenkappe 1 und Halterungsteil 22 von dem Halterungsteil 22 abschraubbar. Das Halterungsteil 22 verbleibt hierzu an der Spritze 28, vergleiche auch Figur 28.

Wie aus Figur 15 hervorgeht, sitzt das Halterungsteil 22 unterseitig unmittelbar auf dem Übergangsbereich des Spritzenhalses 26 in das zylindrische Teil 34 der Spritze 28 auf. Bevorzugt ist die Außenkappe 2 mit einem etwas geringeren Außendurchmesser ausgebildet als das Halterungsteil 22.

Das Halterungsteil 22 bildet einen Halterungskragen 23 mit Innengewinde 24 aus. Das Innengewinde 24 entspricht bevorzugt dem Innengewinde des Halterungsteils 17, wie es im Zusammenhang mit der ersten Ausführungsform beschrieben ist

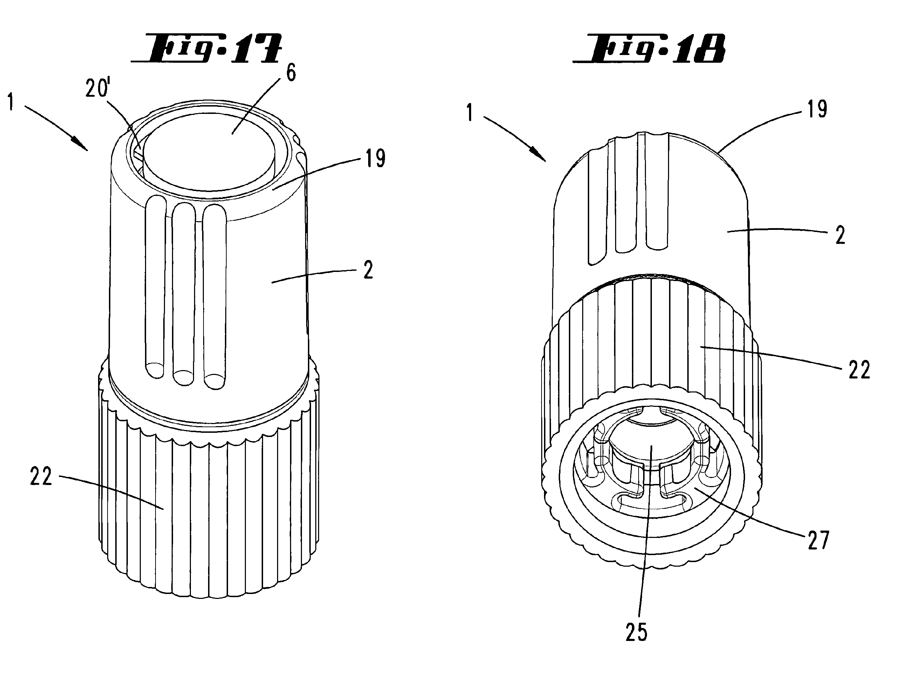

Das Halterungsteil 22 ist mit einer zentralen Durchgangsöffnung 25 ausgebildet mit der es über den die Mündung ausbildenden Hals 26 der Spritze steckbar ist. Hierbei ist an dem Hals 26 eine Verjüngung ausgebildet, bis in welche Halterungsstege 27 der Halterung verfahren werden. Wie insbesondere aus Figur 18 ersichtlich, ist ein Halterungssteg 27 in einer Horizontalprojektion etwa T-förmig gebildet, wobei Enden des T-Querbalkens etwas nach innen vorstehend ausgeformt sind. Unterseitig, also in ihrem in Aufsteckrichtung zunächst mit dem Spritzenhals 26 in Berührung kommenden Bereich, sind sie abgerundet ausgebildet Hierdurch können Sie unter elastischer Verformung - bezogen auf die freikragenden Bereiche des T-Balkens - federnd nach radial außen ausweichen und so bis in den genannten Hinterschnitt hinein verfahren werden. Die oberseitige Kante, wie sich auch aus Figur 21 ergibt, ist dagegen mit einer vergleichsweise scharfen Eckausformung ausgebildet, so dass sich eine gewünschte Verrastung hinter einen an dem Spritzenhals 26 ausgebildeten umlaufenden Absatz 47 ergibt.

Die Zusammenfassung des Halterungsteils 22 mit dem Außenkappenteil 2 und darin befindlicher Innenkappe 3 gemäß den Figuren 17 und 18 kann zur unmittelbaren Steckverbindung mit einer Spritze, wie beschrieben, insbesondere einer Glasspritze, benutzt sein.

### Bezugszeichenliste

| | | | |
|---|---|---|---|
| 1 | Spritzenkappe | 25 | Durchgangsöffnung |
| 2 | Außenkappe | 26 | Spritzenhals |
| 3 | Innenkappe | 27 | Halterungsstege |
| 4 | Abdichtteil | 28 | Spritze |
| 5 | Schraubteil | 29 | Trennfuge |
| 6 | Anzeigelement | 30 | Unterkante |
| 7 | Rastausnehmung | 31 | Oberkante |
| 8 | Innenfläche | 32 | Vorsprung |
| 9 | Eingriffsvorsprung | 33 | Stufe |
| 10 | Rastvorsprung | 34 | zylindrisches Teil (von 28) |
| 11 | Verjüngungsabschnitt | 35 | Kolbenteil |
| 12 | Ausformung | 36 | Nutausformung |
| 13 | Abstandsbereich | 37 | Innengewindegang |
| 14 | Anschlagwand | 38 | Randkante |
| 15 | Gewindegang | 39 | Hinterschnitt |
| 16 | Innengewinde | 40 | Ausformung |
| 17 | Halterung | 41 | Ausnehmung |
| 18 | Auflauframpe | 42 | Luer-Vorsprung |
| 19 | Stirnkante | 43 | Öffnung |
| 20 | Abreißstege | 44 | Durchgriffsöffnungen |
| 20' | Filmgelenk | 45 | Absatz |
| 21 | Auflaufnocken | 46 | Handhabe |
| 22 | Halterungsteil | 47 | Absatz |
| 23 | Halterungskragen | 48 | Vorsprung |
| 24 | Innengewinde | A | Längsachse |

## Patentansprüche

1. Spritzenkappe (1) für eine medizinische Spritze (28), mit zwei zueinander beweglichen Kappenteilen (2, 3), wobei eine Bewegung der Kappenteile (2,3) zueinander bei einem ersten Abnehmen der Spritzenkappe (1) von der Spritze (28) erfolgt und hierbei eine Versetzung eines Anzeigeelementes (6) in eine Anzeigestellung erfolgt, **dadurch gekennzeichnet, dass** das Anzeigeelement (6) zusammen mit den Kappenteilen (2, 3) der Spritzenkappe (1) von der Spritze (28) abnehmbar ist.

2. Spritzenkappe nach Anspruch 1,**dadurch gekennzeichnet, dass** die Spritzenkappe (1) aus einer Außenkappe (2) und einer Innenkappe (3) besteht.

3. Spritzenkappe nach Anspruch 1 oder 2, **dadurch gekennzeichnet, dass** die Kappenteile (2,3) zunächst relativ zueinander verdrehbar sind und am Ende ihrer relativen Verdrehung in eine eine Drehkopplung ergebende Verrastungsstellung überführbar sind, wobei, bevorzugt, die Drehkopplung nicht reversibel ist.

4. Spritzenkappe nach einem der Ansprüche 2 bis 3, **dadurch gekennzeichnet, dass** sich im Zuge der Drehung des Außenkappenteils (2) relativ zu der Innenkappe (3) keine teleskopierende Bewegung ergibt.

5. Spritzenkappe nach einem der Ansprüche 2 bis 4, **dadurch gekennzeichnet, dass** die Außenkappe (2) als fingerhutartige Hülse gestaltet ist, wobei das Anzeigeelement (6) die Stirnfläche oder einen wesentlichen Teil der Stirnfläche bildet.

6. Spritzenkappe nach einem der vorhergehenden Ansprüche, **dadurch gekennzeichnet, dass** sich das Anzeigeelement (6) an der distalen Seite der Spritzenkappe (1) befindet.

7. Spritzenkappe nach einem der Ansprüche 2 bis 6, **dadurch gekennzeichnet, dass** an der Innenkappe (3) ein oder mehrere Gewindegänge (15) ausgebildet sind, zur Zusammenwirkung mit einem ein Gegengewinde aufweisenden Halterungsteil (22) an der Spritze (28), und dass ein Gewindegang (15) oder eine Nutausformung (36) zwischen Abschnitten des Gewindeganges (15) eine Bremsausformung aufweist.

8. Spritzenkappe nach einem der Ansprüche 2 bis 7, **dadurch gekennzeichnet, dass** die Innenkappe (3) aus einem Abdichtteil (4) und einem Schraubteil (5) besteht, wobei, bevorzugt, das Abdichtteil (4) aus einem Gummi- oder Elastomerwerkstoff gebildet ist.

9. Spritzenkappe nach Anspruch 8, **dadurch gekennzeichnet, dass** das Abdichtteil (4) einen Teil einer Außenfläche des Schraubteils (15) bildet, wozu das Abdichtteil (4) bevorzugt radial vorstehend und sich axial erstreckende Ausformungen (40) aufweist.

10. Spritzenkappe nach Anspruch 9, **dadurch gekennzeichnet, dass** die Ausformungen (40) in entsprechenden Durchgriffsöffnungen (44) des Schraubteils (5) einsitzen.

11. Spritzenkappe nach einem der Ansprüche 7 bis 10, **dadurch gekennzeichnet, dass** das Halterungsteil (22) unmittelbar an die Spritze (28) angeformt ist.

12. Spritzenkappe nach einem der Ansprüche 7 bis 11, **dadurch gekennzeichnet, dass** das Halterungsteil (22) gesondert von den Kappenteilen (2, 3) ausgebildet ist.

13. Spritzenkappe nach einem der Ansprüche 7 bis 12, **dadurch gekennzeichnet, dass** das Halterungsteil (22) eine zentrale Durchgangsöffnung, zur Verbindung mit dem Hals (26) eines Spritzenkörpers, vorzugsweise einer Glasspritze, aufweist.

14. Spritzenkappe nach Anspruch 13, **dadurch gekennzeichnet, dass** die Verbindung eine Klemm- und/oder Formschlussverbindung ist.

15. Spritzenkappe nach einem der Ansprüche 7 bis 14, **dadurch gekennzeichnet, dass** an dem Halterungsteil (22) mehrere, in einer horizontalen Projektion T-förmig gebildete Halterungsstege (27) ausgebildet sind.

## Claims

1. A syringe cap (1) for a medical syringe (28), comprising two cap parts (2, 3) that are moveable toward one another, a movement of the cap parts (2, 3) toward one another taking place when the syringe cap (1) is removed for the first time from the syringe (28), and by virtue of this, a displacement of an indicator element (6) into an indicating position takes place, **characterized in that** the indicator element (6) can be removed from the syringe (28) together with the cap parts (2,3) of the syringe cap (1).

2. The syringe cap according to Claim 1, **characterized in that** the syringe cap (1) consists of an outer cap (2) and an inner cap (3).

3. The syringe cap according to Claim 1 or 2, **characterized in that** the cap parts (2, 3) are in first instance twistable relative to one another and at the end of their being twisted relative to one another, they can be transferred into a latching position that results in a twist coupling, whereby, preferably, the twist coupling is not reversible.

4. The syringe cap according to one of Claims 2 to 3, **characterized in that** in the course of the twisting of the outer cap part (2) relative to the inner cap part (3), no telescopic movement results.

5. The syringe cap according one of Claims 2 to 4, **characterized in that** the outer cap (2) is configured as a thimble-like sleeve, the indicator element (6) forming the end face or a substantial part of the end face.

6. The syringe cap according to one of the preceding claims, **characterized in that** the indicator element (6) is located on the distal side of the syringe cap (1).

7. The syringe cap according to one of Claims 2 to 6, **characterized in that** on the inner cap (3) one or a plurality of thread turns (15) are formed for interacting with a mount part (22), comprising a counter-thread, on the syringe (28), and that a thread turn (15), or a groove formation (36) between portions of the thread turn(s), has a brake formation.

8. The syringe cap according to one of Claims 2 to 8, **characterized in that** the inner cap (3) consists of a sealing part (4) and a screw part (5), whereby, preferably, the sealing part (4) is formed from a rubber or elastomer material.

9. The syringe cap according to Claim 8, **characterized in that** the sealing part (4) forms a part of an outer surface of the screw part (15), for which purpose the sealing part (4) preferably has formations (40) that are radially projecting and extend axially.

10. The syringe cap according to Claim 9, **characterized in that** the formations (40) are seated in corresponding passage openings (44) of the screw part (5).

11. The syringe cap according to one of Claims 7 to 10, **characterized in that** the mount part (22) is directly molded onto the syringe (28).

12. The syringe cap according to one of Claims 7 to 11, **characterized in that** the mount part (22) is formed separately from the cap parts (2,3).

13. The syringe cap according to one of Claims 7 to 12, **characterized in that** the mount part (22) has a central through-opening for connecting to the neck (26) of a syringe body, preferably of a glass syringe.

14. The syringe cap according to Claim 13, **characterized in that** the connection is a clamping connection and/or a positive connection.

15. The syringe cap according to one of Claims 7 to 14, **characterized in that** on the mount part (22), a plurality of mount webs (27) are provided, which in a horizontal projection are formed T-shaped.

## Revendications

1. Capuchon de seringue (1) pour une seringue (28) médicale, comportant deux parties de capuchon (2, 3) mobiles l'une par rapport à l'autre, les parties de capuchon (2, 3) effectuant un mouvement l'une par rapport à l'autre lorsque le capuchon de seringue (1) est retiré pour la première fois de la seringue (28), et ledit mouvement induisant un déplacement d'un élément de signalisation (6) dans une position de signalisation, **caractérisé en ce que** l'élément de signalisation (6) peut être retiré de la seringue (28) conjointement avec les parties (2, 3) du capuchon de seringue (1).

2. Capuchon de seringue selon la revendication 1, **caractérisé en ce que** le capuchon de seringue (1) est constitué d'un capuchon extérieur (2) et d'un capuchon intérieur (3).

3. Capuchon de seringue selon la revendication 1 ou 2, **caractérisé en ce que** les parties de capuchon (2, 3) peuvent tourner d'abord l'une par rapport à l'autre et, à la fin de leur rotation relative, peuvent être amenées dans une position d'ancrage engendrant un accouplement rotatif, ledit accouplement rotatif n'étant pas réversible, de préférence.

4. Capuchon de seringue selon l'une des revendications 2 à 3, **caractérisé en ce qu'**au cours de la rotation du capuchon extérieur (2) par rapport au capuchon intérieur (3), il ne se produit aucun mouvement télescopique.

5. Capuchon de seringue selon l'une des revendications 2 à 4, **caractérisé en ce que** le capuchon extérieur (2) est configuré sous la forme d'une gaine en forme de dé à coudre, l'élément de signalisation (6) formant la face frontale ou une partie essentielle de la face frontale.

6. Capuchon de seringue selon l'une des revendications précédentes, **caractérisé en ce que** l'élément de signalisation (6) se situe sur le côté distal du capuchon de seringue (1).

7. Capuchon de seringue selon l'une des revendications 2 à 6, **caractérisé en ce que** sur le capuchon intérieur (3) sont formés un ou plusieurs pas de vis (15) destinés à coopérer avec une partie de retenue (22), munie d'un filetage contraire, sur la seringue (28), et **en ce qu'**un pas de vis (15) ou un façonnage à rainure (36) entre des parties du pas de vis (15) comporte un façonnage de freinage.

8. Capuchon de seringue selon l'une des revendications 2 à 7, **caractérisé en ce que** le capuchon intérieur (3) est constitué d'une partie d'étanchéité (4) et d'une partie de vissage (5), la partie d'étanchéité (4) étant réalisée, de préférence, dans un matériau à base de caoutchouc ou un matériau élastomère.

9. Capuchon de seringue selon la revendication 8, **caractérisé en ce que** la partie d'étanchéité (4) forme une partie d'une surface extérieure de la partie de vissage (15), la partie d'étanchéité (4) comportant à cet effet, de préférence des façonnages (40) saillants dans le sens radial et s'étendant dans le sens axial.

10. Capuchon de seringue selon la revendication 9, **caractérisé en ce que** les façonnages (40) sont insérés dans des ouvertures de passage (44) correspondantes de la partie de vissage (5).

11. Capuchon de seringue selon l'une des revendications 7 à 10, **caractérisé en ce que** la partie de retenue (22) est formée directement sur la seringue (28).

12. Capuchon de seringue selon l'une des revendications 7 à 11, **caractérisé en ce que** la partie de retenue (22) est réalisée séparément des parties de capuchon (2, 3).

13. Capuchon de seringue selon l'une des revendications 7 à 12, **caractérisé en ce que** la partie de retenue (22) comporte une ouverture débouchante centrale pour l'assemblage avec le col (26) d'un corps de seringue, de préférence une seringue en verre.

14. Capuchon de seringue selon la revendication 13, **caractérisé en ce que** l'assemblage est un assemblage par blocage et/ou un assemblage par conjugaison de forme.

15. Capuchon de seringue selon l'une des revendications 7 à 14, **caractérisé en ce que** plusieurs ailettes de retenue (27) en forme de T sur une projection horizontale sont réalisées sur la partie de retenue (22).
